# EUROPÄISCHE PATENTSCHRIFT

(11) **EP 3 326 568 B1**
(45) Veröffentlichungstag und Bekanntmachung des Hinweises auf die Patenterteilung: **22.04.2020**
(21) Anmeldenummer: 17204641.9
(22) Anmeldetag: 13.03.2014
(51) Int. Cl.: A61C 5/62

(54) **SPRITZE**
SYRINGE
SERINGUE

(30) Priorität: 07.05.2013 EP 13166888
(43) Veröffentlichungstag der Anmeldung: 30.05.2018
(62) Teilanmeldung aus: 14710240.4
(73) Patentinhaber: Ivoclar Vivadent AG, 9494 Schaan (LI)
(72) Erfinder: Frank, Müller, 6800 Feldkirch (AT)
(74) Vertreter: Baronetzky, Klaus

(56) Entgegenhaltungen:
- WO-A1-2007/122363
- DE-A1-102005 028 925
- US-A1- 2009 171 311

## Beschreibung

Die Erfindung betrifft eine Spritze, insbesondere für flüssige oder viskose Dentalmaterialien, gemäß den unabhängigen Ansprüchen 1 und 2.

Spritzen für flüssige oder viskose Dentalmaterialien sind seit langem bekannt. Als Beispiel hierfür sei die bereits etwas neuere US 4,863,072 erwähnt. Bei dieser Spritze lässt sich ein Kolben zum Ausdrücken von Dentalmaterial in ein Spritzengehäuse einschrauben, um so eine gesteuerte und fein dosierbare Abgabe des Dentalmaterials zu ermöglichen.

Für eine raschere Abgabe des Dentalmaterials ist bei dieser Lösung auch eine Zwei-Hand-Bedienung möglich, bei welcher das Spritzengehäuse mit einer Hand bzw. mit zwei Fingern dieser Hand gegriffen wird und der dortige Betätigungsknauf, der in Form einer Scheibe ausgebildet ist, rasch gedreht wird.

In diesem Zusammenhang wird es jedoch als unbefriedigend empfunden, dass der Zeitaufwand bei rascher Betätigung beträchtlich ist und ein rasches Betätigen durch eine schnelle manuelle Drehung erfordert, die als unangenehm empfunden wird.

Eine raschere Abgabe des dortigen zu injizierenden Materials ist praktisch nur durch noch schnelleres Verdrehen der Scheibe möglich.

Eine weitere Spritze ist aus der WO 2007/122363 A1 bekannt.

Zahlreiche weitere Spritzen sind bereits vorgeschlagen worden. Als Beispiel sei die DE 199 00 792 C1 genannt, gemäß welcher eine Dosierung durch Verdrehen eines Rändelrads erfolgen soll. Diese Lösung ist recht kompliziert und erfordert auch passgenau hergestellte Einzelteile, was zu entsprechend hohen Herstellungskosten führt.

Demgegenüber liegt der Erfindung die Aufgabe zugrunde, eine Spritze gemäß dem Oberbegriff von Anspruch 1 zu schaffen, die trotz der Verwendung eines kostengünstigen Herstellverfahrens eine rasche Spritzenentleerung, aber bei Bedarf auch eine feine Dosierung ermöglicht.

Diese Aufgabe wird erfindungsgemäß durch Anspruch 1 und Anspruch 2 gelöst. Vorteilhafte Weiterbildungen ergeben sich aus den Unteransprüchen.

Erfindungsgemäß besonders günstig ist es, dass das Spritzengehäuse, in welchem der Kolben geführt ist, über einen Teil seiner Länge fest, also gegenüber dem Kolben unnachgiebig, ausgebildet ist, und über einen weiteren Teil seiner Länge verformbar ist, wobei bevorzugt auch dieser Teil des Spritzengehäuses vom Kolben durchdringbar ist. Durch die Kombination dieser Teile lässt sich nach Wahl des Benutzers das Dentalmaterial rasch ausdrücken, indem in an sich bekannter Weise auf den Kolben gedrückt und dieser translatorisch bewegt wird, oder aber zur Feindosierung der verformbare Teil zusammengedrückt wird, so dass Dentalmaterial ausgedrückt wird.

Es versteht sich, dass für das Wirksamwerden des Ausdrückens der Schubwiderstand des Kolbens, also die Haftreibung des Kolbens im Spritzengehäuse, größer als der Fließwiderstand des Dentalmaterials durch die Auslasskanüle hindurch ist. Dies ist bei flüssigen Dentalmaterialien ohnehin automatisch so, und zwar auch dann, wenn der Kolben sich bereits in dem verformbaren Teil des Spritzengehäuses befindet, also eine nahezu vollständige Entleerung bereits stattgefunden hat.

Auch bei viskosen Dentalmaterialien ist es günstig, wenn der Schiebewiderstand des Kolbens in dem Spritzengehäuse, also insofern die Haftreibung beim stillstehenden Kolben, an die Viskosität angepasst ist, also bei viskoserem Dentalmaterial eine größere Haftreibung eingestellt wird als bei flüssigerem Material.

Die Einstellung kann in beliebiger geeigneter Weise über die Wahl der Abmessungen des Kolbens einerseits und des Spritzengehäuses andererseits vorgenommen bzw. eingestellt werden. Bevorzugt weist das Spritzengehäuse einen Innendurchmesser auf, der etwas, beispielsweise um 0,2 mm, größer als der Außendurchmesser des zylindrischen Kolbens ist. In an sich bekannter Weise weist der Kolben an seinem vorderen, also der Auslasskanüle benachbarten, Ende eine Ringnut auf. In dieser ist in ebenfalls an sich bekannter Weise ein O-Ring aufgenommen. Der O-Ring überragt in radialer Richtung den Außendurchmesser des Kolbens beispielsweise um 0,4 mm. Hierdurch erfolgt die Abdichtung zwischen der Wand des Spritzengehäuses und dem Kolben, dessen O-Ring beim Einführen in das Spritzengehäuse zusammengedrückt wird und über den Verlauf der Schiebebewegung des Kolbens in gleicherweise zusammengedrückt verbleibt.

Beispielsweise kann die Ringnut eine Breite von 2 mm und eine radiale Tiefe von 1,6 mm aufweisen. Wenn ein O-Ring mit entsprechendem Durchmesser und einer Stärke von 2 mm eingesetzt wird, springt dieser dementsprechend um 2 mm - 1,6 mm = 0,4 mm nach außen vor und wird beim Einführen in das Spritzengehäuse um 0,2 mm zusammengedrückt. Die Größe des Anpressdrucks entscheidet über den Schiebewiderstand des Kolbens, aber auch über die Haftreibung des Kolbens in dem Spritzengehäuse.

Um nun einen größeren Wert für die Haftreibung zu erzielen, kann entweder ein O-Ring mit einer Stärke von 2,1 mm eingesetzt werden, oder die Tiefe der Ringnut beispielsweise auf 1,5 mm verringert werden. In beiden Fällen lässt sich das erwünschte Ergebnis erzielen, dass beim Zusammendrücken des vorderen Teils des Spritzengehäuses Dentalmaterial aus der Auslasskanüle ausgedrückt wird, ohne dass das Ausdrücken dazu führen würde, dass sich der Kolben rückwärts bewegt.

Besonderes Augenmerk wird auf die Führung des Kolbens im vorderen, also nachgiebigen Teil des Spritzengehäuses gelegt. Beispielsweise ist es möglich, den nachgiebigen Teil des Spritzengehäuses nach der Art einer Griffmulde lediglich an zwei aneinander gegenüberliegenden Bereichen des Spritzengehäuses zu realisieren, und das Spritzengehäuse im Übrigen formstabil bis zur Auslasskanüle erstrecken zu lassen.

Die Dimensionierung erfolgt jedenfalls so, dass ein "Überströmen", also ein Bewegen von Dentalmaterial über die Dichtung des Kolbens hinweg, sicher vermieden ist. Auch hier gilt, dass die relativen Abmessungen des Kolbens einerseits und des Spritzengehäuses andererseits an die Viskosität des Dentalmaterials anpassbar ist. Bei flüssigem Dentalmaterial müssen typischerweise keine besonderen Vorkehrungen getroffen werden, da das flüssige Dentalmaterial sich durch die Auslasskanüle leicht ausdrücken lässt, wenn die einander gegenüberliegenden Bereiche des verformbaren Teils des Spritzengehäuses zusammengedrückt werden.

Bei viskoserem Dentalmaterial kann es günstig sein, wenn die Elastizität des leicht verformbaren Bereichs des Spritzengehäuses ausgenutzt wird, um den Anpressdruck zu erhöhen. Hierzu kann das Spritzengehäuse dort - beispielsweise durch Wärmeschrumpfen - mit einem Untermaß gefertig sein, so dass die Dichtung dort dann beispielsweise nicht nur um 0,2 mm, sondern um 0,3 mm zusammengedrückt werden muss, wenn der Kolben in den verformbaren Bereich des Spritzengehäuses eindringen soll.

Erfindungsgemäß günstig ist es, wenn der verformbare Bereich des Spritzengehäuses der Auslasskanüle benachbart ist. Hierdurch wird zunächst erreicht, dass die Feindosierung auch dann zur Verfügung steht, wenn der Kolben sich über mehr als die Hälfte zum Auslassende hin bewegt hat, also die Sprite beispielsweise halb entleert ist. Der verformbare Bereich kann beispielsweise das vordere Viertel oder das vordere Fünftel des zylindrischen Teils des Spritzengehäuses abdecken. Das Spritzengehäuse lässt sich auch dann zusammendrücken, wenn der Kolben sich bereits etwas in den verformbaren Bereich hineinerstreckt hat, indem nämlich lediglich der Auslasskanüle benachbarte Teil des verformbaren Bereichs dann zusammengedrückt wird.

Es versteht sich, dass gerade bei einem viskosen Dentalmaterial typischerweise kein Zurückfedern des verformbaren Bereichs nach Druckbetätigung erfolgt. Dies ist günstig, denn dadurch wird ein Ansaugen durch die Auslasskanüle verhindert, die ansonsten Verunreinigungen erzeugen würde. Vielmehr wird der verformbare Bereich erst beim weiteren Betätigen der Druckstange des Kolbens wieder auf Nennmaß gebracht, also auseinandergedrückt.

Insofern sind besondere Federeigenschaften des verformbaren Bereichs des Spritzengehäuses nicht erforderlich oder erwünscht, hingegen sehr wohl dessen begrenzte Verformbarkeit.

Die Verformbarkeit des vorderen Bereichs des Spritzengehäuses lässt sich in weiten Bereichen an die Erfordernisse anpassen und nach Belieben des Anwenders gestalten. So ist es beispielsweise möglich, ein aus einem harten Kunststoff bestehendes Spritzengehäuse als Zylinder zu fertigen, der vorne zwei Durchgriffsausnehmungen hat. In dieses Außengehäuse lässt sich dann ein Innengehäuse des Spritzengehäuses einschieben und an der gewünschten Stelle befestigen. Das Innengehäuse deckt dann die Aussparungen des Außengehäuses ab und an dieser Stelle ist das Spritzengehäuse dann nachgiebig und verformbar. Der Kolben kann das Innengehäuse dann über seine ganze zylindrische Länge durchtreten, und an dem Innengehäuse ist dann typischerweise die Auslasskanüle einstückig angeformt.

Diese Lösung hat den Vorteil, dass der Kolben bis zum vorderen Ende des zylinderförmigen Teils des Innengehäuses führbar ist, so dass keine oder ganz geringe Rückstände des vergleichsweise teuren Dentalmaterials in der Spritze bei deren Entleerung verbleiben.

Alternativ und mit den gleichen Vorteilen lässt sich das Außengehäuse ebenfalls bis ganz nach vorne führen, und durch Ausgestaltung von Zungen das Außengehäuse selbst verformbar realisieren. Durch Druck auf die Zungen des Außengehäuses wird dann das Innegehäuse zusammengedrückt und ermöglicht das feindosierte Abgeben von Dentalmaterialien.

Gemäß einer weiteren Ausgestaltung der Erfindung ist der Kolben in an sich bekannter Weise mit einer Drehspindel verbunden. Das Spritzengehäuse weist ein Innengewinde auf, und die Drehspindel führt den Kolben durch die Drehbewegung des Drehgriffs der Drehspindel zum Ausdrücken des Dentalmaterials. Diese Lösung erlaubt dem Grunde nach bereits ein feineres Dosierien als Spritzen, bei denen ein Kolben rein translatorisch geführt ist.

Dennoch ist es günstig, einen verformbaren Bereich des Spritzengehäuses vorzusehen, der sich nach vorne, also zur Auslasskanüle hin anschließt und an welchem durch leichtes zusammendrücken der Wandung des Spritzengehäuses Dentalmaterial ausgedrückt wird. Dieses Feindosieren kann rasch und gezielt und auch mit visueller und sensorischer Kontrolle erfolgen.

Gemäß einer weiteren, besonders bevorzugten Ausgestaltung ist der verformbare Bereich in dem Verlauf der Auslasskanüle selbst angeordnet. Bei dieser Ausgestaltung ist der verformbare Bereich nach der Art einer Pumpe ausgebildet, weist also typischerweise zwei Rückschlagventile auf, eins vor und eins nach der Membran der Pumpe, so dass beim Zusammendrücken des dort angeordneten verformbaren Bereichs das Material nach vorne gedrückt wird, und zwar unabhängig von der Haftreibung des Kolbens im zylindrischen Teil des Spritzengehäuses.

Der Vorteil dieser Ausgestaltung ist, dass der durch Druck auf den elastischen Teil 50 im Inneren der Spritze wirkende Druck das Dentalmaterial durch das vordere Ventil 82 aus der Kanüle 20 fördert.

Beim Nachlassen des Fingerdrucks auf den elastischen Bereich 50 erfolgt der Druckausgleich im Bereich des Pumpengehäuses 84 durch Ansaugen von Dentalmaterial durch das hintere, näher zum Kolben 22 hin platzierte Ventil 80. Dadurch ist eine Spritze nach dieser Bauform nach jeder Betätigung automatisch wieder förderfähig, ohne den Stößel 42 zusätzlich betätigen zu müssen. Die Spritze ist insofern selbstansaugend.

Die Kolbenreibung kann dadurch sehr gering gehalten werden. Beispielsweise kann hierzu die Reibung zwischen dem Kolben und der Innenwand der Spritze auf geringe Werte eingestellt werden, was insgesamt den Betätigungskomfort erhöht.

In weiterer vorteilhafter Ausgestaltung ist es vorgesehen, den verformbaren Bereich oder mindestens einen Teil des verformbaren Bereich des Spritzengehäuses durchsichtig oder durchscheinend auszubilden. Durch diese an sich bekannte Maßnahme lässt sich leicht eine visuelle Kontrolle des Zustands der Spritze, aber auch des verbleibenden Dentalmaterials realisieren.

Weitere Vorteile, Einzelheiten und Merkmalen ergeben sich aus der nachfolgenden Beschreibung von Ausführungsbeispielen anhand der Zeichnungen.

Es zeigen:
- Fig. 1: eine leicht schematisierte, erläuterungshalber beigefügte Schnittansicht einer Ausführungsform einer Spritze 10;
- Fig. 2: ein Teil der Spritze 10 gemäß Fig. 1, in einer seitlichen Draufsicht; und
- Fig. 3: eine Ausführungsform einer erfindungsgemäßen Spritze 10, unter Darstellung eines Teils dieser Spritze 10, nämlich eines Teils der Auslasskanüle 20;
- Fig. 4: eine schematische erläuterungshalber beigefügte Ansicht einer Ausführungsform einer Spritze;
- Fig. 5: eine schematische erläuterungshalber beigefügte Ansicht einer Ausführungsform einer Spritze;
- Fig. 6: eine schematische erläuterungshalber beigefügte Ansicht einer Ausführungsform einer Spritze; und
- Fig. 7: eine schematische Ansicht einer weiteren Ausführungsform einer erfindungsgemäßen Spritze.

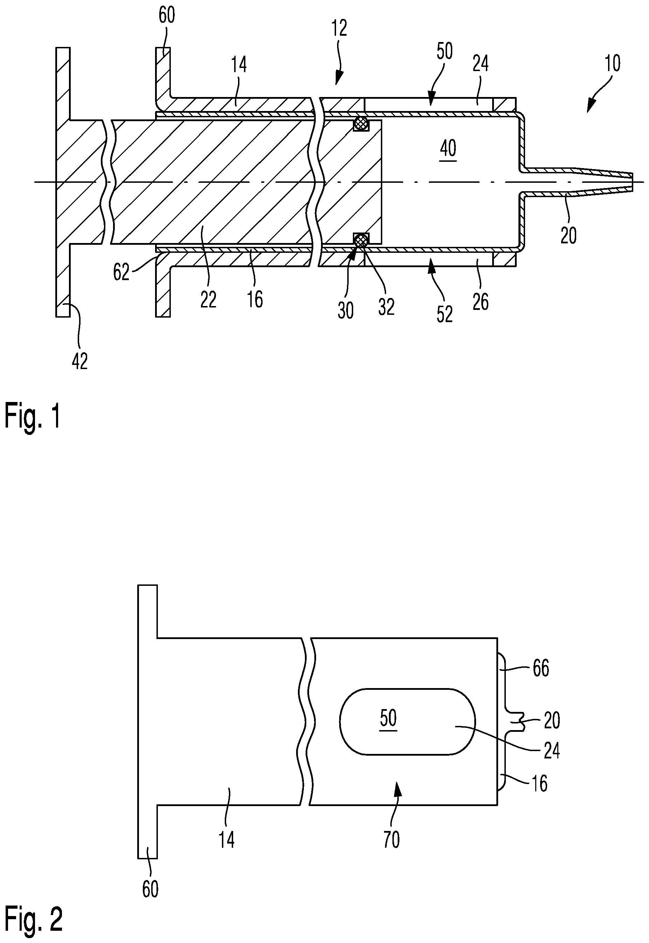

Die in Fig. 1 dargestellte Spritze 10 weist ein Spritzengehäuse 12 auf, das in dem dargestellten Ausführungsbeispiel zweiteilig ausgebildet ist.

Das Spritzengehäuse 12 weist ein Außengehäuse 14 und ein Innengehäuse 16 auf. Das Außengehäuse 14 hat eine vergleichsweise große Wandstärke von beispielsweise 1 mm oder 2 mm. Es ist aus einem härter eingestellten Kunststoff ausgebildet.

Das Innengehäuse 16 kann demgegenüber eine dünnere Wandstärke von beispielsweise 0,2 bis 0,3 mm aufweisen. Sowohl aufgrund der geringen Wandstärke als auch aufgrund der Materialwahl, nämlich eines etwas weicher eingestellten Kunststoffs, ist das Innengehäuse 16 an sich nachgiebig ausgebildet und insofern verformbar. Es ist jedoch in seinem überwiegenden Teil von dem Außengehäuse 14 abgestützt. Der Innendurchmesser des Au ßengehäuses entspricht dem Au ßendurchmesser des Innengehäuses 16 oder ist um wenige µ unterschiedlich.

Am vorderen Ende des Spritzengehäuses 12 ist eine Auslasskanüle 20 ausgebildet. Die Auslasskanüle 20 ist in an sich bekannter Weise konisch zulaufend ausgebildet, so dass die Strömungsquerschnittsfläche eingangsseitig der Auslasskanüle 20 beispielsweise das Zwanzigfache, bevorzugt etwa das Sechsfache des Strömungsquerschnitts am Austrittsende der Auslasskanüle 20 beträgt. Der Innendurchmesser des Einlassendes der Auslasskanüle 20 beträgt dementsprechend etwa das Zweieinhalbfache des Innendurchmessers am Auslassende der Auslasskanüle 20.

In ebenfalls an sich bekannter Weise ist die Auslasskanüle 20 in Strömungsverbindung mit dem zylindrischen Teil des Spritzengehäuses 12 verbunden. Der zylindrische Teil endet entweder in einem Konus mit einem sehr stumpfen Konuswinkel oder flach, wie es in Fig. 1 dargestellt ist, um so möglichst geringe Verluste an Dentalmaterial sicherzustellen. Bis zum Ende des zylindrischen Teils des Spritzengehäuses 12 ist ein Kolben 22 einschiebbar, um das vorher in der Spritze 10 aufgenommene Dentalmaterial auszudrücken.

In dem dargestellten Ausführungsbeispiel ist die Auslasskanüle 20 und das Zylinderende des zylindrischen Teils des Spritzengehäuses 12 einstückig mit dem Innengehäuse 16 ausgebildet. Es versteht sich, dass anstelle dessen aber auch eine beliebige andere geeignete Ausgestaltung wählbar ist.

Das Außengehäuse 14 weist zwei einander gegenüberliegende Durchbrechungen 24 und 26 auf. Mit diesen Durchbrechungen ist gewährleistet, dass die Außenseite des Innengehäuses 16 an dieser Stelle, die an der Außlasskanüle 20 benachbart ist, frei zugänglich ist.

Der Kolben 22 weist einen Durchmesser auf, der dem Innendurchmesser des Innengehäuses 16 im Wesentlichen entspricht und um weniges, beispielsweise 0,1 mm, kleiner als dieser ist. An seinem vorderen, also dem der Auslasskanüle 20 zugewandten Ende kann beispielsweise eine Ringnut 30 ausgebildet sein, die einen Dichtungsring 32 aufnimmt. Der Au ßendurchmesser des Kolbens 22 mit eingelegtem Dichtungsring 32 ist etwas größer als der Innendurchmesser des Innengehäuses 16, beispielsweise um 0,2 mm oder 0,3 mm. Der Dichtungsring 32 ist in an sich bekannter Weise elastisch kompressibel und erzeugt eine Abdichtung des Innenraums 40 der Spritze 10, der mit Dentalmaterial gefüllt ist.

Anstelle dessen kann aber auch eine elastische Kappe mit angeformten Dichtlippen vorgesehen sein.

Die Abdichtung durch den Dichtungsring 32 ist so stark, dass bei Druck auf den Kolben 22 über die Greifhandhabe 42 des Kolbens 22 Dentalmaterial aus dem Innenraum 40 über die Auslasskanüle 20 ausgedrückt wird, aber der Dichtungsring 32 gegen das Innengehäuse 16 abdichtet.

Auch bei Druck auf die freiliegenden Flächen 50 und 52 des Innengehäuses 16 unter den Durchbrechungen 24 und 26 strömt kein Dentalmaterial an dem Dichtungsring 32 vorbei. Beim Druck auf diese Flächen 50 und 52 wird der Kolben 22 auch nicht nach hinten gedrückt, da die Reibung zwischen dem Dichtungsring 32 und dem Innengehäuse 16 ausreichend groß ist, um dies zu verhindern. Vielmehr wird hierdurch feinfühlig Dentalmaterial aus der Auslasskanüle 20 ausgedrückt und abgegeben.

In dem dargestellten Ausführungsbeispiel weist das Außengehäuse 14 eine an seinem rückwärtigen Ende einen umlaufenden Kragen 60 auf. Für das Betätigen des Kolbens 22 nimmt der Benutzer die Spritze 10 zwischen Zeigefinger und Mittelfinger, so dass Zeigefinger und Mittelfinger von der vorderen Seite der Spritze 10 aus an den Kragen 60 anliegen. Der Daumen der gleichen Hand des Benutzers liegt an der Greifhandhabe 42 an. Hierdurch lässt sich leicht in an sich bekannter Weise eine Einhandbedienung für die schnelle Abgabe von Dentalmaterial realisieren.

Bei Feindosierung wird der Griff seitens des Benutzers geändert. Die Spritze 10 wird nunmehr zwischen Daumen und Zeigefinger ausschließlich an den Flächen 50 und 52 gefasst, während der rückwärtige Teil der Spritze 10 einschließlich der Greifhandhabe 42 an dem Handballen anliegt und gegebenenfalls von dem Ringfinger stabilisiert wird. Beim Druck zwischen Zeigefinger und Daumen des Benutzers lässt sicht nun so feinfühlig Dentalmaterial abgeben, und der Benutzer ist hierbei bei der Abgabe des Dentalmaterials mit den gut steuerbaren Fingern Daumen und Zeigefinger ausgesprochen nah am Abgabeort, so dass die Dosierung als auch die positionelle Steuerung präzise und genau möglich ist.

Insofern lässt sich erfindungsgemäß trotz der Kombination der Abgabe mit größerer Menge als auch der Feindosierung eine Einhandbedienung realisieren, ohne dass die Herstellung der erfindungsgemäßen Spritze 10 besonders aufwändig wäre.

Für die Fixierung des Innengehäuses 16 an dem Außengehäuse 14 können diese an dem rückwärtigen Ende 62 miteinander verschweißt sein. Es ist aber auch möglich, dass dort das Innengehäuse 16 einen weiteren Kragen ausbildet, der ein Durchdrücken durch das Au ßengehäuse 14 verhindert.

Die Auslasskanüle 20 kann in an sich bekannter Weise außen und im rückwärtigen Bereich ein Außengewinde tragen, dass für den Eingriff in ein Innengewinde einer Verschlusskappe bestimmt ist. Derartige Verschlusskappen und Systeme sind seit langem und an sich bekannt.

Aus Fig. 2 ist ersichtlich, in welcher Weise sich die Durchbrechung 24 greiffreundlich erstreckt und zugleich die Fläche 50 des Innengehäuses 16 freigibt. Wie ersichtlich ist, ragt das Innengehäuse 16 etwas nach vorne aus dem Außengehäuse vor, nachdem die radiale Abstützung dort durch die Stirnwand 66 des Innengehäuses 16 übernommen wird.

Die Dimensionierung der Materialstärken und Störmungswiderstände ist so bemessen, dass beim Ausdrücken des Dentalmaterials aus dem Innenraum 40 über die Betätigung des Kolbens 22 auch im Bereich der Durchbrechungen 24 und 26 kein Überströmen über den Dichtungsring 32 erfolgt. Hierzu sind die Durchbrechungen 24 und 26 ausreichend klein gewählt, so dass eine Abstützung auch im vorderen Bereich über das Außengehäuse 14 besteht. Alternativ kann auch das Außengehäuse selbst an dieser Stelle beweglich gehalten sein, beispielsweise über Schlitze, die ein Zusammendrücken partiell erlauben, oder auch über einseitige oder beidseitige Hebel, die außen an der Spritze 10 angebracht sind und dem Zusammendrücken dienen.

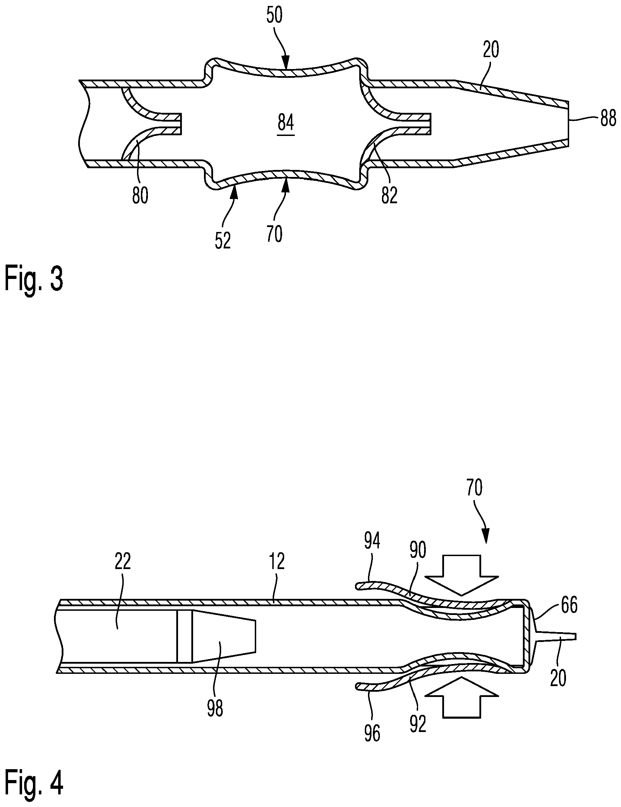

Eine Ausgestaltung der Erfindung ist aus Fig. 3 ersichtlich. Bei dieser Lösung ist der verformbare Teil des Spritzengehäuses 12 in die Auslasskanüle 20 verlagert (oder in das vordere Ende der Spritze nahe der Auslasskanüle). Bei der Ausführungsform gemäß Fig. 1 und Fig. 2 ist der verformbare Bereich 70 der Auslasskanüle 20 benachbart, während er bei der Ausführungsform gemäß Fig. 3 Teil dieser ist. Dementsprechend ist der gesamte zylindrische Teil des Spritzengehäuses 12 formstabil und durch die üblichen Kräfte nicht oder nicht wesentlich verformbar. Der verformbare Bereich 70 des Spritzengehäuses 12 weist bei dieser Lösung Flächen 50 und 52 auf, die aufeinander zu zusammendrückbar sind. Sie sind in dem dargestellten Ausführungsbeispiel fingerfreundlich gestaltet, können aber sowohl konkav als auch konvex ausgebildet sein. Stromab und stromauf dieses verformbaren Bereichs 50 sind je Rückschlagventile 80 und 82 ausgebildet.

Beim Zusammendrücken der Flächen 50 und 52 entsteht Druck im Innenraum 84 des verformbaren Bereichs 70. Da das Rückschlagventil 80 schließt, öffnet sich das Rückschlagventil 82, und Dentalmaterial kann über das Auslassende 88 der Auslasskanüle 20 abgegeben werden. Beim Loslassen der Flächen 20 und 22 möchte das Material der Auslasskanüle 20 wieder in den Ausgangszustand zurückkehren, nachdem es elastisch ist. Aufgrund der Wirkung des Rückschlagventils 82 kann das Dentalmaterial jedoch aber nicht zurückströmen, so dass sich das Rückschlagventil 80 öffnet und Material nachströmen kann.

Dieses wird über den Innenraum 40 nachgesaugt, oder durch Druck auf die Greifhandhabe 42 des Kolbens 22 nachgeschoben.

Es versteht sich, dass bei dieser Lösung gegebenenfalls auch auf das Rückschlagventil 82 verzichtet werden kann, wenn ein Rückströmen des Dentalmaterials aus dem Auslassende unkritisch sein sollte.

Die in Fig. 4 dargestellte Ausführungsform zeigt eine Spritze 10, bei der der Bereich 70 des Spritzengehäuses 12 verformbar ist. Die Verformung erfolgt über in dem der Auslasskanüle 20 benachbarten Bereich 70 vorgesehene elastische Seitenwände des Spritzengehäuses 12. Es sind Hebel 90 und 92 an dem vorderen Ende des zylindrischen Teils des Spritzengehäuses 12 der Stirnwand 66 benachbart angebracht. An dieser Stelle ist jeder Hebel 90 und 92 in beliebiger geeigneter Weise beweglich mit dem Spritzengehäuse 12 verbunden. In dem dargestellten Ausführungsbeispiel ist jeder Hebel im wesentlichen S-förmig geschwungen und, so dass er im Bereich 70 auf das Spritzengehäuse 12 drücken kann, wenn an den fernen Enden 94 und 96 je Druck auf den betreffenden Hebel 90 und 92 ausgeübt wird.

Mit dieser Ausgestaltung lässt sich noch feinfühliger die Abgabe von Dentalmaterial über die Auslasskanüle einstellen, denn der Hebel 90 - und gleichermaßen der Hebel 92 - wirkt untersetzend, so dass eine Bewegung der Enden 94 und 96 zu einer geringeren Bewegung im Bereich 70 führt.

In dem dargestellten Ausführungsbeispiel sind die Hebel 90 und 92 als einarmige Hebel ausgebildet, wobei der Lastarm je kürzer als der Kraftarm ist.

In dem dargestellten Ausführungsbeispiel ist ferner der Kolben 22 mit einem Konus 98 an seiner der Auslasskanüle 20 zugewandten Spitze versehen. Diese Lösung ermöglicht es, den Kolben 22 bis an die Stirnwand 66 anliegend nach vorne zu schieben, aber dennoch den verformbaren Bereich 70 zu verformen. Eine Feindosierung ist damit bis zur vollständigen Entleerung des Dentalmaterials möglich.

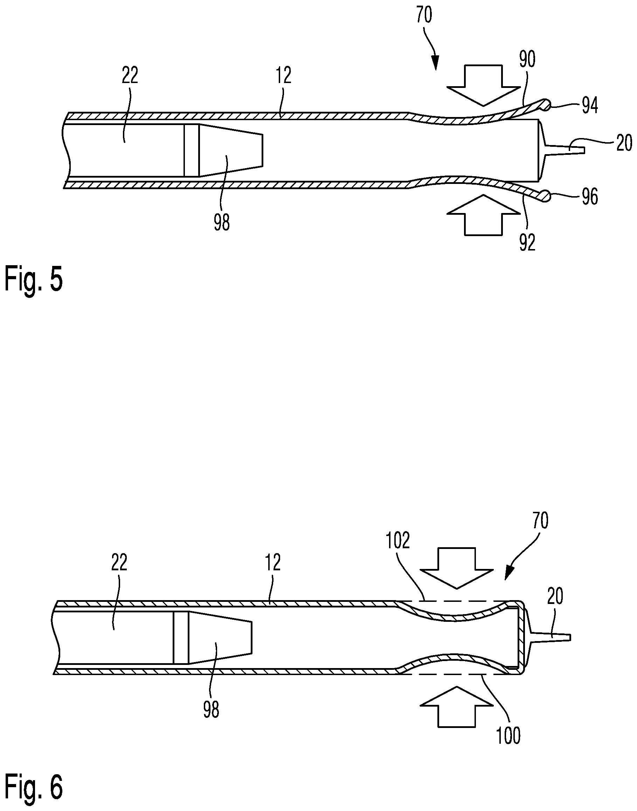

Eine ähnliche Ausgestaltung ist aus Fig. 5 ersichtlich. Auch hier sind Hebel 90 und 92 vorgesehen, über die untersetzend Druck auf den verformbaren Bereich 70 des Spritzengehäuses 12 ausübbar ist, wobei der Bereich 70 wiederum der Auslasskanüle 20 benachbart ist.

Auch hier weist der Kolben 22 an der Spitze einen Konus 98 auf, der störungsfrei in den verformbaren Bereich 70 eindringen kann.

Im Unterschied zu der Ausführungsform gemäß Fig. 4 ist bei der Ausführungsform gemäß Fig. 5 jeder Hebel 90 und 92 an dem der Auslasskanüle 20 gegenüberliegenden Ende des verformbaren Bereich 70 angelenkt. Dementsprechend weisen die Enden 94 und 96 nach vorne, also zur Auslasskanüle 20 hin. Auch hier lässt sich eine Untersetzung und eine entsprechend feinfühlige Dosierung der Materialabgabe erzielen.

Bei der Ausführungsform gemäß Fig. 6 ist es vorgesehen, den verformbaren Bereich 70 unmittelbar in das Spritzengehäuse einzuarbeiten. Das Spritzengehäuse ist bei dieser Ausführungsform dementsprechend nicht zweiwandig wie bei den Ausführungsformen gemäß Fig. 1 und Fig. 2 sondern einwandig. Der formstabile Bereich des Spritzengehäuses 12 weist im Grunde Durchbrechungen entsprechend den Durchbrechungen 24 und 26 aus Fig. 1 auf. Diese sind jedoch durch elastische Einsätze 100 und 102 geschlossen, so dass auch hier eine Feindosierung in der bereits beschriebenen Weise möglich ist.

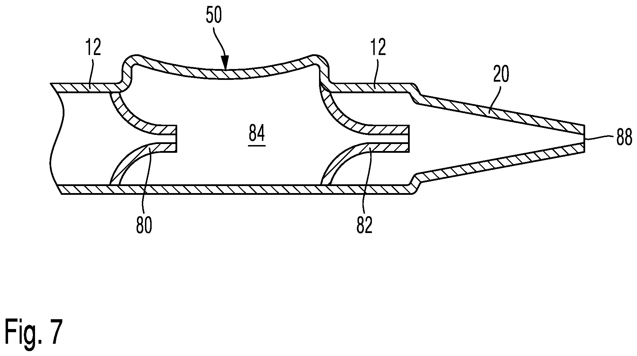

Während in Fig. 1 die beispielsweise aus Fig. 3 oder Fig. 7 ersichtliche Pumpe nicht ersichtlich ist, versteht es sich, dass die dort dargestellte Ausführungsform ohne Weiteres auch eine entsprechende Pumpe aufweisen kann.

Die in Fig. 7 dargestellte Pumpe ist für die Einfingerbetätigung vorgesehen. Im Unterschied zu der Ausführungsform gemäß Fig. 3 ist die Betätigung durch einseitigen Druck auf die Fläche 50 möglich, wobei es sich versteht, dass die gegenüberliegende Fläche, die zylindrisch ausgebildet ist, formstabiler ist.

Ein besonderer Vorteil dieser Ausgestaltung ist es, dass mit den zwei Ventilen, die den Innenraum 84 umgeben, besonders gut Druck erzeugt werden kann. Auch bei viskoseren Materialien, die exprimiert werden sollen, lässt sich so feinfühlig die Materialabgabe dosieren.

Es ist möglich, die elastische Wand im Bereich der Fläche 50 in Form eines markanten Druckknopfes auszugestalten; dies macht das System dem Anwender sogar selbsterklärend.

Auch wenn im Rahmen der vorliegenden Ausführungsbeispiele die Ventile 80 und 82 als sogenannte Entenschnabelventile ausgestaltet sind, versteht es sich, dass beliebige andere Ventilbauformen ebenso möglich sind, beispielsweise Membranventile oder Flatterventile, und im Grunde jede Ventilart, die in Strömungsrichtung automatisch öffnet und gegen die Strömungsrichtung automatisch sperrt.

## Patentansprüche

1. Spritze für viskose oder flüssige Dentalmaterialien, mit einem Spritzengehäuse (12), in welchem ein Kolben (22) in an sich bekannter Weise schiebebeweglich geführt ist, und mit einer Auslasskanüle (20), die an dem Spritzengehäuse (12) angebracht ist, wobei
ein der Auslasskanüle (20) benachbarter Bereich (50, 70) verformbar ist und eine Pumpe aufweist und ein von der Auslasskanüle (20) entfernter Bereich des Spritzengehäuses (12) formstabil ausgebildet ist und den Kolben (22) schiebebeweglich führt, und
dass die Auslasskanüle (20) ein Rückschlagventil (82) aufweist, das das Einsaugen von Luft bei Entlastung des Kolbens (22) und/oder des verformbaren Bereichs verhindert.

2. Spritze für viskose oder flüssige Dentalmaterialien, mit einem Spritzengehäuse (12), in welchem ein Kolben (22) in an sich bekannter Weise schiebebeweglich geführt ist, und mit einer Auslasskanüle (20), die an dem Spritzengehäuse (12) angebracht ist, wobei
ein in der Auslasskanüle (20) angeordneter Bereich (50, 70) verformbar ist und eine Pumpe aufweist und ein von der Auslasskanüle (20) entfernter Bereich des Spritzengehäuses (12) formstabil ausgebildet ist und den Kolben (22) schiebebeweglich führt, und
dass die Auslasskanüle (20) ein Rückschlagventil (82) aufweist, das das Einsaugen von Luft bei Entlastung des Kolbens (22) und/oder des verformbaren Bereichs verhindert.

3. Spritze nach Anspruch 1 oder 2, **dadurch gekennzeichnet, dass** die Pumpe zwei Rückschlagventile (80,82) aufweist, eins stromab und eins stromauf des verformbaren Bereichs (50, 70).

4. Spritze nach einem der vorhergehenden Ansprüche, **dadurch gekennzeichnet, dass** der verformbare Bereich (50, 70) des Spritzengehäuses (12) Flächen (50, 52) aufweist, die aufeinander zu zusammendrückbar sind.

5. Spritze nach Anspruch 1, **dadurch gekennzeichnet, dass** die Pumpe zur Einfingerbetätigung ausgebildet ist, wobei die Betätigung durch einseitigen Druck auf eine Fläche (50) der Pumpe möglich ist, wobei die gegenüberliegende zylindrische Fläche formstabiler ausgebildet ist.

6. Spritze nach Anspruch 5, **dadurch gekennzeichnet, dass** die elastische Wand im Bereich der Fläche (50) nach der Art eines markanten Druckknopfes ausgestaltet ist.

7. Spritze nach einem der Ansprüche 3 bis 6, **dadurch gekennzeichnet, dass** die Rückschlagventile (80, 82) als Entenschnabelventile (80, 82) ausgebildet sind.

8. Spritze nach einem der Ansprüche 3 bis 6, **dadurch gekennzeichnet, dass** die Rückschlagventile (80, 82) als Membranventile ausgebildet sind.

9. Spritze nach einem der Ansprüche 3 bis 6, **dadurch gekennzeichnet, dass** die Rückschlagventile (80, 82) als Flatterventile ausgebildet sind.

10. Spritze nach einem der Ansprüche 1 oder 3 bis 9, **dadurch gekennzeichnet, dass** der verformbare Bereich eine Länge zwischen 10 und 50%, bevorzugt zwischen 15 und 30% und insbesondere zwischen 20 und 25% der Länge des Spritzengehäuses (12) einnimmt, und/oder dass der verformbare Bereich des Spritzengehäuses (12) eine Länge zwischen 1cm und 3cm, insbesondere etwa 2cm hat.

11. Spritze nach einem der vorhergehenden Ansprüche, **dadurch gekennzeichnet, dass** der Kolben (22) aus dem Spritzengehäuse (12) entnehmbar und in dieses einführbar ist.

## Claims

1. A syringe for viscous or liquid dental materials, comprising a syringe housing (12) in which a piston (22) is slidably guided in a manner known per se, and an outlet cannula (20) which is attached to the syringe housing (12), wherein a region (50, 70) adjacent to the outlet cannula (20) is deformable and comprises a pump, and a region of the syringe housing (12) remote from the outlet cannula (20) is dimensionally stable and slidably guides the piston (22), and
that the outlet cannula (20) comprises a check valve (82) which prevents air from being aspirated when relieving the piston (22) and/or the deformable region.

2. The syringe for viscous or liquid dental materials comprising a syringe housing (12) in which a piston (22) is slidably guided in a manner known per se, and an outlet cannula (20) which is attached to the syringe housing (12), wherein a region (50, 70) located in the outlet cannula (20) is deformable and comprises a pump, and a region of the syringe housing (12) remote from the outlet cannula (20) is dimensionally stable and slidably guides the piston (22), and that the outlet cannula (20) comprises a check valve (82) which prevents air from being aspirated when relieving the piston (22) and/or the deformable region.

3. The syringe according to claim 1 or 2, **characterized in that** the pump comprises two check valves (80, 82), one downstream and one upstream of the deformable region (50, 70).

4. The syringe according to one of the preceding claims,
**characterized in that** the deformable region (50, 70) of the syringe housing (12) comprises surfaces (50, 52) which are compressible towards each other.

5. The syringe according to claim 1, **characterized in that** the pump is designed for single-finger actuation, the actuation being enabled by unilateral pressure on one surface (50) of the pump, the opposite cylindrical surface being designed to be more dimensionally stable.

6. The syringe according to claim 5, **characterized in that** the elastic wall in the region of the surface (50) is designed in the manner of a distinctive push-button.

7. The syringe according to one of claims 3 to 6, **characterized in that** the check valves (80, 82) are formed as duck-bill valves (80, 82).

8. The syringe according to one of claims 3 to 6, **characterized in that** the check valves (80, 82) are formed as diaphragm valves.

9. The syringe according to one of claims 3 to 6, **characterized in that** the check valves (80, 82) are formed as flutter valves.

10. The syringe according to one of the claims 1 or 3 to 9, **characterized in that**
the deformable region occupies a length between 10 and 50%, preferably between 15 and 30% and especially between 20 and 25% of the length of the syringe housing (12), and/or in that the deformable region of the syringe housing (12) has a length between 1cm and 2cm.

11. The syringe according to one of the preceding claims, **characterized in that** the piston (22) is removable and insertable from and into the syringe housing (12).

## Revendications

1. Seringue pour matériaux dentaires visqueux ou liquides, avec un boîtier de seringue (12) dans lequel un piston (22) est guidé de manière coulissante de manière connue en soi,.et avec une canule de sortie (20) qui est fixée sur le boîtier de seringue (12), où une zone (50, 70) adjacente à la canule de sortie (20) est déformable et comprend une pompe, et une zone du boîtier de seringue (12) éloignée de la canule de sortie (20) est conçue indéformable et guide le piston (22) de manière coulissante, et en ce que la canule de sortie (20) comprend un clapet anti-retour (82) qui empêche l'aspiration d'air lorsque le piston (22) et/ou la zone déformable sont déchargés.

2. Seringue pour matériaux dentaires visqueux ou liquides, avec un boîtier de seringue (12) dans lequel un piston (22) est guidé de manière coulissante de manière connue en soi, et avec une canule de sortie (20) qui est fixée sur le boîtier de seringue (12), où une zone (50, 70) dans la canule de sortie (20) est déformable et comprend une pompe, et une zone du boîtier de seringue (12) éloignée de la canule de sortie (20) est conçue indéformable et guide le piston (22) de manière coulissante, et en ce que la canule de sortie (20) comporte un clapet anti-retour (32) qui empêche l'aspiration d'air lorsque le piston (22) et/ou la zone déformable sont déchargés.

3. Seringue selon la revendication 1 ou 2, **caractérisée en ce que** la pompe comprend deux clapets anti-retour (80, 82), un en aval et un en amont de la partie déformable (50, 70).

4. Seringue selon l'une des revendications précédentes, **caractérisée en ce que** la partie déformable (50, 70) du boîtier de seringue (12) présente des surfaces (50, 52) qui sont compressibles l'une vers l'autre.

5. Seringue selon la revendication 1, **caractérisée en ce que** la pompe est conçue pour être actionnée par un seul doigt, où l'actionnement est possible par une pression unilatérale sur une surface (50) de la pompe, où la surface cylindrique opposée est conçue pour être plus stable dimensionnellement.

6. Seringue selon la revendication 5, **caractérisée en ce que** la paroi élastique dans la zone de la surface (50) est conçue à la manière d'un bouton-poussoir notable.

7. Seringue selon l'une des revendications 3 à 6, **caractérisée en ce que** les clapets anti-retour (80, 82) sont conçus comme des valves à bec de canard (80, 82).

8. Seringue selon l'une des revendications 3 à 6, **caractérisée en ce que** les clapets anti-retour (80, 82) sont conçus comme des clapets à membrane.

9. Seringue selon l'une des revendications 3 à 6, **caractérisée en ce que** les clapets anti-retour (80, 82) sont conçus comme des clapets flottants.

10. Seringue selon l'une des revendications 1 ou 3 à 9, **caractérisée en ce que** la partie déformable occupe une longueur comprise entre 10 et 50%, de préférence entre 15 et 30% et en particulier entre 20 et 25% de la longueur du boîtier de seringue (12), et/ou **en ce que** la partie déformable du boîtier de seringue (12) a une longueur comprise entre 1 cm et 3 cm, en particulier environ 2 cm.

11. Seringue selon l'une des revendications précédentes, **caractérisée en ce que** le piston (22) est amovible et insérable dans le boîtier de la seringue (12).
